# EUROPÄISCHE PATENTSCHRIFT

(11) **EP 0 936 891 B1**
(45) Veröffentlichungstag und Bekanntmachung des Hinweises auf die Patenterteilung: **09.01.2002**
(21) Anmeldenummer: 98936216.5
(22) Anmeldetag: 18.06.1998
(51) Int. Cl.: A61F 5/01

(54) **ORTHESENGELENK**
ORTHOSIS ARTICULATION
ARTICULATION POUR ORTHESE

(30) Priorität: 03.09.1997 DE 29715794 U
(43) Veröffentlichungstag der Anmeldung: 25.08.1999
(73) Patentinhaber: Otto Bock HealthCare GmbH, 37115 Duderstadt (DE)
(72) Erfinder: WAGNER, Helmut, D-37115 Duderstadt (DE)
(74) Vertreter: Gramm, Werner, Prof. Dipl.-Ing.
(86) Internationale Anmeldenummer: DE9801673
(87) Internationale Veröffentlichungsnummer: WO9911206

(56) Entgegenhaltungen:
- EP-A- 0 016 268
- DE-U- 29 613 843
- US-A- 2 591 373
- US-A- 4 502 472
- US-A- 5 460 599

## Beschreibung

Die Erfindung betrifft ein Orthesengelenk, insbesondere Kniegelenk für ein Beinteil, bestehend aus einem Gelenkoberteil und einem mit diesem verschwenkbar verbundenen Gelenkunterteil sowie aus einer manuell lösbaren Keilsperre, deren im Gelenkoberteil verschiebbar geführter Sperrkeil in seiner Sperrstellung in eine am Gelenkunterteil vorgesehene Sperrnut eingreift und aus dieser Sperrstellung gegen die Wirkung einer Feder in eine Entriegelungsstellung verschiebbar ist.

Eine derartige Ausführungsform lässt sich der US-PS 2,591,373 entnehmen. Hier besteht das Gelenkoberteil aus einer Hülse in Form eines aus einem Blech gebogenen Hohlkammerprofils, in dessen oberen, mantelseitig geschlossenen Abschnitt das untere Ende einer oberen Orthesenschiene eingeschoben und mit der Hülse vernietet ist. Die Schwenkverbindung zwischen dem hülsenförmigen Gelenkoberteil und dem Gelenkunterteil erfolgt über einen Stift oder Niet. Der Sperrkeil ist in einer länglichen Ausnehmung in der oberen Orthesenschiene verschiebbar geführt, wobei diese längliche Ausnehmung von dem hülsenförmigen Gelenkoberteil auf drei Seiten umgriffen ist. Die längliche Ausnehmung erstreckt sich in Längsrichtung der oberen Orthesenschiene, wobei der Sperrkeil in seiner Sperrstellung in bezogen auf die Achse der Gelenkverschwenkung etwa tangentialer Richtung in die genannte Sperrnut eingreift.

Die EP 0 016 268 A1 offenbart ebenfalls ein Orthesengelenk bestehend aus einem Gelenkoberteil und einem mit diesem verschwenkbar verbundenen Gelenkunterteil sowie aus einer manuell lösbaren Keilsperre, deren im Gelenkoberteil verschiebbar geführter Sperrkeil in seiner Sperrstellung in bezogen auf die Verschwenkachse etwa radialer Richtung in eine am Gelenkunterteil vorgesehene Sperrnut eingreift und aus dieser Sperrstellung gegen die Wirkung einer Feder in eine Entriegelungsstellung verschiebbar ist.

Der Erfindung liegt die Aufgabe zugrunde, die Funktionalität des eingangs beschriebenen Orthesengelenkes zu verbessern.

Ausgehend von dem eingangs beschriebenen Orthesengelenk wird diese Aufgabe erfindungsgemäß dadurch gelöst, dass die Schwenkverbindung zwischen Gelenkoberteil und Gelenkunterteil über eine Gelenkschraube erfolgt, und dass das Gelenkoberteil in einer senkrecht auf der Gelenkschraube stehenden Ebene in einen Grundkörper und einen diesen zumindest teilweise abdeckenden Deckel zweigeteilt ist, und dass der Sperrkeil in miteinander korrespondierenden nutenförmigen Ausfräsungen in den einander zugewandten Innenseiten des Grundkörpers und Deckels geführt ist und in seiner Sperrstellung in bezogen auf die Gelenkschraube etwa radialer Richtung in die Sperrnut eingreift.

Die erfindungsgemäß vorgesehene Zweiteilung des Gelenkoberteils führt im Vergleich zu dem eingangs beschriebenen Stand der Technik nicht nur zu einer stabileren Konstruktion sondern auch zu einer erleichterten Zugänglichkeit zu der Keilsperre, die durch bloßes Lösen des Deckels vom Grundkörper freigelegt wird. Außerdem führen die miteinander korrespondierenden nutenförmigen Ausfräsungen zu einer präziseren Führung des Sperrkeils im Vergleich zu der eingangs beschriebenen Ausführungsform, wo die Sperrkeil-Führung im Wesentlichen durch umgebördelte Flansche des hülsenförmigen Gelenkoberteils gebildet und überdies wegen eines nach außen offenen Sperrkeil-Führungsschlitzes einer Verschmutzungsgefahr ausgesetzt ist. Das erfindungsgemäße Eingreifen des Sperrkeils in etwa radialer Richtung führt zu einer stabileren und damit sicheren Arretierung im Vergleich zu einer tangentialen Anordnung. Die erfindungsgemäß vorgesehene gekapselte Sperrkeil-Führung verhindert eine Verschmutzung im Ver schiebebereich des Sperrkeils und verbessert dadurch die Funktionalität. Die den Sperrkeil beidseitig übergreifenden Ausfräsungen bilden eine sehr exakte Führung und erlauben daher eine zuverlässige, verkantungsfreie Verschiebung des Sperrkeils.

Eine hinsichtlich des Kraftaufwandes besonders günstige Betätigung der Keilsperre ist dann gewährleistet, wenn ein manuell zu betätigendes Zugelement, vorzugsweise eine Perlonschnur unmittelbar an dem Sperrkeil in dessen Verschieberichtung angreift.

Eine besonders stabile, weitgehend gegen Verschmutzung geschützte Gelenkverbindung ist erfindungsgemäß dadurch erreichbar, dass das Gelenkunterteil mit einem scheibenförmig ausgebildeten, die Gelenkschraube aufnehmenden und die zumindest eine Sperrnut aufweisenden Abschnitt sandwichartig zwischen Grundkörper und Deckel des Gelenkoberteils verschwenkbar geführt ist.

Weitere Vorteile ergeben sich aus der Darstellung und Erläuterung eines Ausführungsbeispiels.

In der Zeichnung ist eine als Beispiel dienende Ausführungsform der Erfindung dargestellt. Es zeigen:
- **Figur 1 -**: ein Orthesenkniegelenk in äußerer Seitenansicht;
- **Figur 2 -**: die Darstellung gemäß Figur 1 in Stirnansicht;
- **Figur 3 -**: die Ausführungsform gemäß Figur 1 in perspektivischer Darstellung;
- **Figur 4 -**: die Darstellung gemäß **Figur 3** in Rückansicht;
- **Figur 5 -**: in vergrößertem Maßstab die Darstellung gemäß Figur 1 mit gestrichelt eingezeichneter Keilsperre;

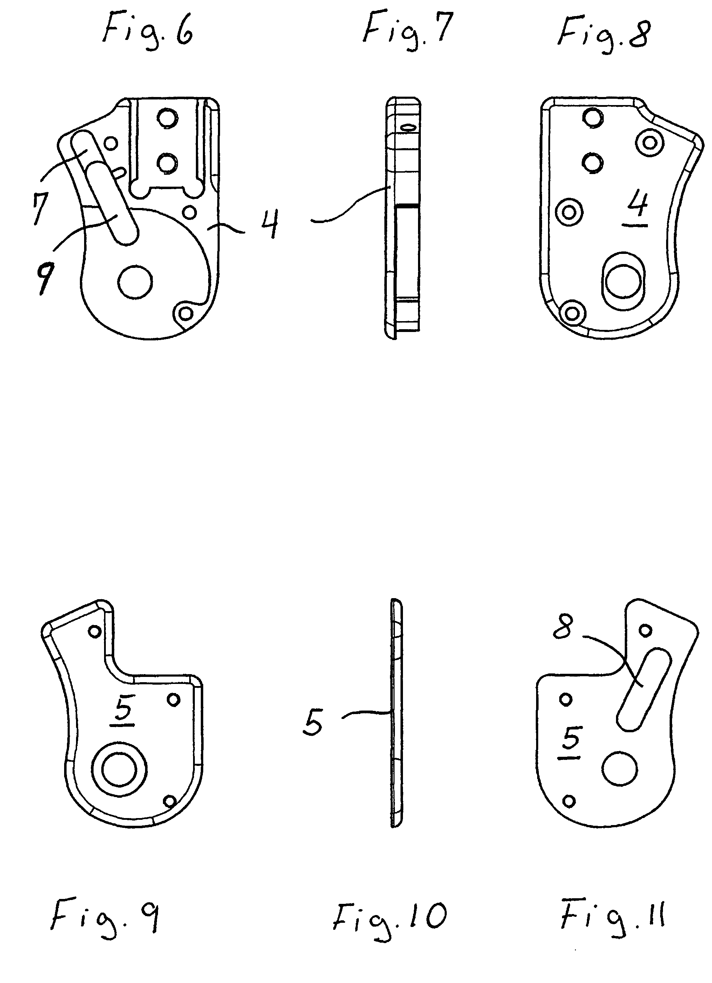

- **Figur 6 -**: die Innenseite eines das Gelenkoberteil bildenden Grundkörpers;
- **Figur 7 -**: den Grundkörper gemäß **Figur 6** in Stirnansicht;
- **Figur 8 -**: die Rückseite des Grundkörpers gemäß **Figur 6**;
- **Figur 9 -**: die Innenseite eines den Grundkörper gemäß den **Figuren 6 bis 8** abdeckenden Deckels;
- **Figur 10 -**: den Deckel gemäß **Figur 9** in Stirnansicht und
- **Figur 11 -**: die Rückseite des Deckels gemäß **Figur 9**.

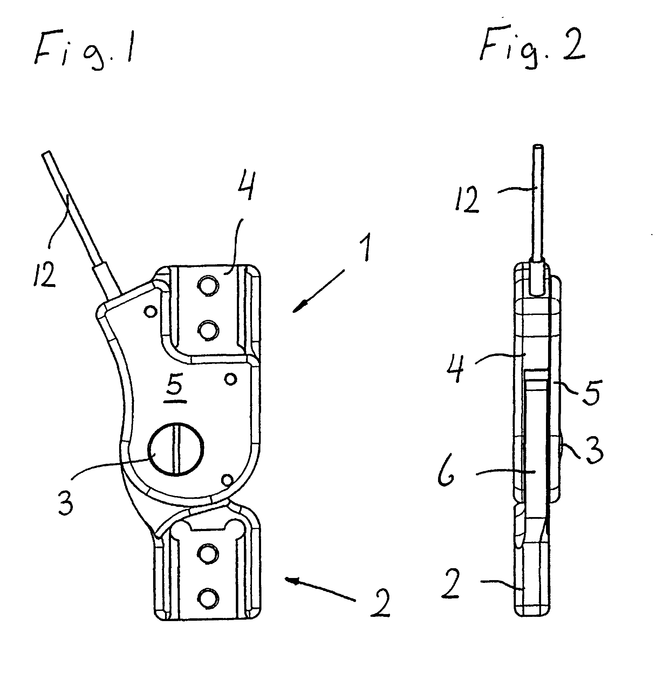

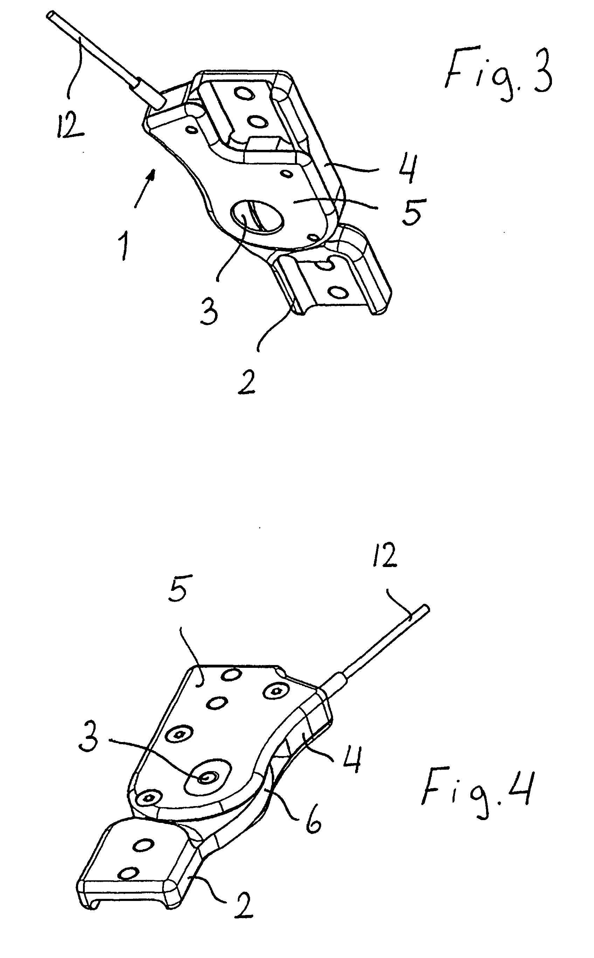

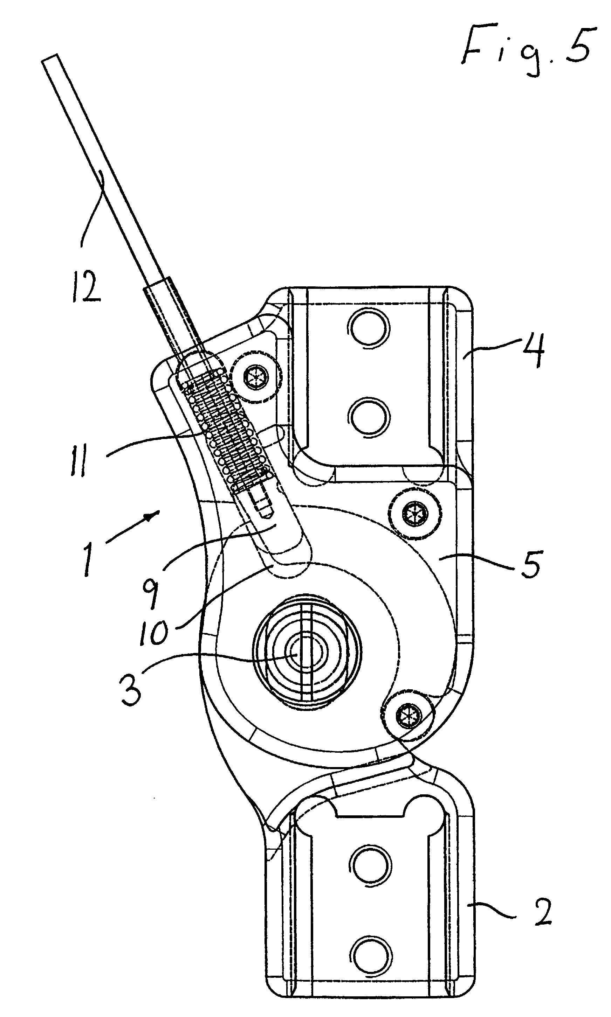

Die Figuren 1 bis 5 zeigen ein Orthesengelenk, das als Kniegelenk für ein Beinteil ausgebildet ist. Dieses Kniegelenk besteht im wesentlichen aus einem Gelenkoberteil 1 und einem Gelenkunterteil 2, die über eine Gelenkschraube 3 verschwenkbar miteinander verbunden sind.

Das Gelenkoberteil 1 ist in einer senkrecht auf der Gelenkschraube 3 stehenden Ebene in einen Grundkörper 4 und einen diesen teilweise abdeckenden Deckel 5 zweigeteilt. In zusammengebautem Zustand ist zwischen Grundkörper 4 und Deckel 5 sandwichartig ein scheibenförmig ausgebildeter, die Gelenkschraube 3 aufnehmender Abschnitt 6 des Gelenkunterteils 2 verschwenkbar geführt.

In der Innenseite des Grundkörpers 4 und in der Innenseite des Deckels 5 ist jeweils eine nutenförmige Ausfräsung 7, 8 vorgesehen, die in zusammengebauten Zustand zwischen sich einen verschiebbaren Sperrkeil 9 aufnehmen. Dieser Sperrkeil 9 greift in seiner Sperrstellung in - bezogen auf die Gelenkschraube 3 - etwa radialer Richtung in eine am oberen Außenrand des Abschnitts 6 des Gelenkunterteils 2 vorgesehene Sperrnut 10 ein und ist aus dieser Sperrstellung gegen die Wirkung einer Druckfeder 11 in eine Entriegelungsstellung verschiebbar. Diese Entriegelung erfolgt durch manuelle Betätigung und zwar durch Ziehen an einer Perlonschnur 12, die unmittelbar an dem Sperrkeil 9 in dessen Verschieberichtung, also in Richtung der länglichen Ausfräsungen 7, 8 angreift.

## Patentansprüche

1. Orthesengelenk, insbesondere Kniegelenk für ein Beinteil, bestehend aus einem Gelenkoberteil (1) und einem mit diesem verschwenkbar verbundenen Gelenkunterteil (2) sowie aus einer manuell lösbaren Keilsperre (9, 10, 11, 12), deren im Gelenkoberteil (1) verschiebbar geführter Sperrkeil (9) in seiner Sperrstellung in eine am Gelenkunterteil (2) vorgesehene Sperrnut (10) eingreift und aus dieser Sperrstellung gegen die Wirkung einer Feder (11) in eine Entriegelungsstellung verschiebbar ist, **dadurch gekennzeichnet, dass** die Schwenkverbindung zwischen Gelenkoberteil (1) und Gelenkunterteil (2) über eine Gelenkschraube (3) erfolgt, und dass das Gelenkoberteil (1) in einer senkrecht auf der Gelenkschraube (3) stehenden Ebene in einen Grundkörper (4) und einen diesen zumindest teilweise abdeckenden Deckel (5) zweigeteilt ist, und dass der Sperrkeil (9) in miteinander korrespondierenden nutenförmigen Ausfräsungen (7, 8) in den einander zugewandten Innenseiten des Grundkörpers (4) und Deckels (5) geführt ist und in seiner Sperrstellung in bezogen auf die Gelenkschraube (3) etwa radialer Richtung in die Sperrnut (10) eingreift.

2. Orthesengelenk nach Anspruch 1, **dadurch gekennzeichnet, dass** ein manuell zu betätigendes Zugelement, vorzugsweise eine Perlonschnur (12) unmittelbar an dem Sperrkeil (9) in dessen Verschieberichtung angreift.

3. Orthesengelenk nach Anspruch 1 oder 2, **dadurch gekennzeichnet, daß** das Gelenkunterteil (2) mit einem scheibenförmig ausgebildeten, die Gelenkschraube (3) aufnehmenden und die zumindest eine Sperrnut (19) aufweisenden Abschnitt (6) sandwichartig zwischen Grundkörper (4) und Deckel (5) des Gelenkoberteils (1) verschwenkbar geführt ist.

## Claims

1. Orthotic joint, in particular a knee joint for a leg part, comprising a joint upper part (1) and a joint lower part (2) pivotally connected to the latter as well as a manually releasable wedge-type lock (9, 10, 11, 12), of which the locking wedge (9) guided displaceably in the joint upper part (1) engages in its locking position into a locking groove (10) provided in the joint lower part (2) and is displaceable counter to the action of a spring (11) out of said locking position into an unlocking position, **characterized in that** the swivel connection between joint upper part (1) and joint lower part (2) is effected by means of a joint screw (3), and that the joint upper part (1) is divided in a plane perpendicular to the joint screw (3) into two parts, namely a basic body (4) and a cover (5) which at least partially covers the latter, and that the locking wedge (9) is guided in mutually corresponding groove-shaped countersunk portions (7, 8) in the opposing inner surfaces of basic body (4) and cover (5) and engages in its locking position in an, in relation to the joint screw (3), approximately radial direction into the locking groove (10).

2. Orthotic joint according to claim 1, **characterized in that** a manually operable pulling element, preferably a Perlon cord (12), acts directly upon the locking wedge (9) in the direction of displacement of the latter.

3. Orthotic joint according to claim 1 or 2, **characterized in that** the joint lower part (2) is pivotally guided by means of a disk-shaped portion (6), which receives the joint screw (3) and comprises the at least one locking groove (19), in a sandwich-like manner between basic body (4) and cover (5) of the joint upper part (1).

## Revendications

1. Articulation pour orthèse, en particulier articulation du genou pour une pièce de jambe, constituée d'une pièce articulaire supérieure (1) et d'une pièce articulaire inférieure (2) reliée de manière pivotante avec la pièce articulaire supérieure, ainsi que d'un blocage par clavette pouvant être libéré manuellement (9, 10, 11, 12) et possédant, guidée à coulisse dans la pièce articulaire supérieure (1), une clavette de blocage (9) qui, dans sa position de blocage, s'engage dans une rainure de blocage (10) prévue sur la pièce articulaire inférieure (2) et peut coulisser de cette position de blocage à une position de déverrouillage à l'encontre de l'effet d'un ressort (11), **caractérisée en ce que** la liaison pivotante entre la pièce articulaire supérieure (1) et la pièce articulaire inférieure (2) est réalisée par l'intermédiaire d'une vis d'articulation (3), **en ce que** la pièce articulaire supérieure (1) est divisée en deux, suivant un plan perpendiculaire à la vis d'articulation (3), en un corps de base (4) et un couvercle (5) qui le recouvre au moins partiellement, et **en ce que** la clavette de blocage (9) est guidée dans des fraisages (7, 8) en forme de rainure qui se correspondent mutuellement dans les côtés intérieurs, tournés l'un vers l'autre, du corps de base (4) et du couvercle (5), et dans sa position de blocage la clavette de blocage s'engage dans la rainure de blocage (10) en direction sensiblement radiale par rapport à la vis d'articulation (3).

2. Articulation pour orthèse selon la revendication 1, **caractérisée en ce qu'**un élément de traction à actionnement manuel, de préférence un fil en perlon (12), s'accroche directement à la clavette de blocage (9) dans la direction de coulissement de cette dernière.

3. Articulation pour orthèse selon la revendication 1 ou 2, **caractérisée en ce que** la pièce articulaire inférieure (2) est guidée en pivotement par une partie configurée en forme de disque (6), montée en sandwich entre le corps de base (4) et le couvercle (5) de la pièce articulaire supérieure (1), cette partie (6) recevant la vis d'articulation (3) et présentant l'au moins une rainure de blocage (19).
